(19) Europäisches Patentamt
European Patent Office
Office européen des brevets

(11) EP 4 101 452 A1

(12) **EUROPEAN PATENT APPLICATION**
published in accordance with Art. 153(4) EPC

(43) Date of publication:
**14.12.2022 Bulletin 2022/50**

(21) Application number: **20917330.1**

(22) Date of filing: **02.06.2020**

(51) International Patent Classification (IPC):
***A61K 31/4706*** *(2006.01)* ***A61K 31/5415*** *(2006.01)*
***A61P 31/14*** *(2006.01)* ***A61P 11/00*** *(2006.01)*

(86) International application number:
**PCT/CN2020/093989**

(87) International publication number:
**WO 2021/155651 (12.08.2021 Gazette 2021/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.02.2020 CN 202010078808**

(71) Applicant: **Academy of Military Medical Sciences**
**Beijing 100850 (CN)**

(72) Inventors:
- **ZHONG, Wu**
  **Beijing 100850 (CN)**
- **XIAO, Gengfu**
  **Beijing 100850 (CN)**
- **HU, Zhihong**
  **Beijing 100850 (CN)**
- **WANG, Manli**
  **Beijing 100850 (CN)**
- **ZHANG, Leike**
  **Beijing 100850 (CN)**
- **CAO, Ruiyuan**
  **Beijing 100850 (CN)**
- **LI, Wei**
  **Beijing 100850 (CN)**
- **FAN, Shiyong**
  **Beijing 100850 (CN)**
- **ZHOU, Xinbo**
  **Beijing 100850 (CN)**
- **LI, Song**
  **Beijing 100850 (CN)**

(74) Representative: **Inspicos P/S**
**Agern Allé 24**
**2970 Hørsholm (DK)**

Remarks:
The complete document including Reference Table(s) and the Sequence Listing(s) can be downloaded from the EPO website

(54) **USE OF 4-AMINOQUINOLINE COMPOUND IN TREATMENT OF CORONAVIRUS INFECTION**

(57) Disclosed are hydroxychloroquine or chloroquine, or a geometric isomer thereof, or a pharmaceutically acceptable salt thereof, and/or a solvate thereof, and/or a hydrate thereof, and a pharmaceutical composition containing the above-mentioned compound, and the use thereof in the treatment of diseases or infections caused by SARS-CoV-2.

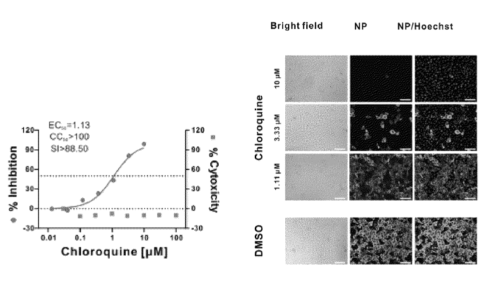


A B

Figure 1

EP 4 101 452 A1

## Description

The invention is based on and claims the benefit of priority from Chinese application No. 202010078808.7, filed on February 3, 2020, the disclosure of which are incorporated herein by reference in its entirety.

## Technical Field

The present application relates to use of hydroxychloroquine represented by Formula I or chloroquine represented by Formula II, a geometric isomer, a pharmaceutically acceptable salt, a solvate and/or a hydrate thereof, and a pharmaceutical composition comprising the above-mentioned compound in the treatment of a SARS-CoV-2 infection.

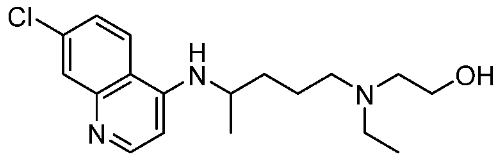

I

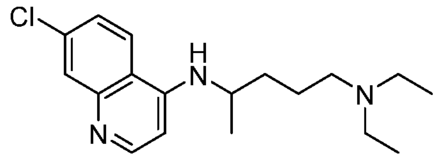

II.

## Background Art

Hydroxychloroquine (compound of Formula I) has a chemical name of 2-[[4-[(7-chloro quinolin-4-yl)amino] pentyl]ethylamino]-ethanol; chloroquine (compound of Formula II) has a chemical name of N',N'-Diethyl-N-4-(7-chloroquinolin-4-yl)-1,4-pentanediamine.

Both the two compounds are 4-aminoquinoline antimalarials, which have functions such as immunosuppression, anti-inflammation, reduction of ultraviolet-induced reaction, sun protection, anti-thrombosis, influence on porphyrin metabolism, anti-hyperlipidemia, anti-proliferation, and anti-pathogen microorganisms. Currently, hydroxychloroquine sulfate is clinically used for the treatment of rheumatoid arthritis, juvenile chronic arthritis, discoid and systemic lupus erythematosus, and skin lesions caused or exacerbated by sunlight. Chloroquine is clinically used to treat falciparum malaria, vivax malaria and quartan malaria that are sensitive to chloroquine, it can also be used for suppressive prevention of malaria symptoms, and it can also be used for the treatment of extraintestinal amebiasis, connective tissue disease, photosensitivity diseases (e.g., erythema solare), etc.

Hydroxychloroquine was artificially synthesized in 1944 and was initially used for antimalarial treatment. In 1955, it was used for the treatment of systemic lupus erythematosus (SLE). Compared with chloroquine phosphate, because hydroxychloroquine has a hydroxyl group, it retains the original efficacy of chloroquine, and has a toxicity half lower than that of chloroquine, thereby having better safety.

Studies have found that in addition to the above known clinical effects, hydroxychloroquine exhibits activities against flavivirus and dengue virus in vitro.

The $IC_{50}$ of hydroxychloroquine for DENV-2 infected cells is as follows: $10.1\pm1.6$ $\mu$M (A549 cells); $12.9\pm4.2$ $\mu$M (Hepa1-6 cells); $12.9\pm1.9$ $\mu$M (WS-1 cells). In the A549 cell line, hydroxychloroquine at 50 to 80 $\mu$M can reduce DENV-2 virus titer by 100 times. In addition, hydroxychloroquine can inhibit the DENV-2 to infect J774A.1 macrophages ($IC_{50}$ = 9.7 $\pm$ 1.3 $\mu$M).

Studies have found that hydroxychloroquine is an inhibitor of Zika virus protease NS2B-NS3 and has an inhibitory constant Ki of $92.34\pm11.91\mu$M for Zika virus protease NS2B-NS3. Hydroxychloroquine has the effect of resisting ZIKV infection in pregnant mice, the dosage of which is 40 mg/kg/day, and which is intraperitoneally administrated from the first day after ZIKV infection. The results indicate that compared to the mice treated with PBS, the placenta of mice treated with hydroxychloroquine has a lower level of ZIKV infection.

The 2019 novel coronavirus (2019-nCoV) is a new coronavirus strain that has never been found in humans before. On February 11, 2020, the International Committee on Taxonomy Viruses (ICTV) announced that the official name of 2019 novel Coronavirus (2019-nCoV) is called severe acute respiratory syndrome coronavirus 2 (SARS-CoV-2). On the same day, the World Health Organization (WHO) announced that the official name of the disease caused by this virus is COVID-19. The symptoms of SARS-CoV-2 virus infection are mainly pneumonia, and can be divided into simple infection, mild pneumonia, severe pneumonia, acute respiratory distress syndrome, sepsis, septic shock and so on according to the severity of disease. Patients with simple infection may have non-specific symptoms, such as fever, cough, sore throat, nasal congestion, fatigue, headache, muscle pain or discomfort, and the elderly and immunosuppressed people may have atypical symptoms. Patients with mild pneumonia mainly have cough, dyspnea and polypnea. Severe pneumonia can be seen in adolescents, adults or children, and the main symptoms of which include increased

breathing frequency, severe respiratory failure or dyspnea, central cyanosis, drowsiness, unconsciousness or convulsion, gasp, etc. The lung images of acute respiratory distress syndrome are bilateral ground glass shadows, which cannot be completely explained by effusion, lobar exudation or atelectasis or lung mass shadows, and the main symptom of which is pulmonary edema. Patients with sepsis often have fatal organ dysfunction, and the most critical patients are those with septic shock, and they may have a high probability of death.

At present, the 2019 novel coronavirus (SARS-CoV-2) infection is mainly treated with supportive therapy in clinic, and no specific antiviral drug is available.

**Contents of the Invention**

The purpose of the present application is to discover a drug with an antiviral activity against a coronavirus, especially SARS-CoV-2, which can be used for the treatment of a relative disease caused by infection thereof, for example, simple infection (such as fever, cough and sore throat), pneumonia, acute respiratory infection or severe acute respiratory infection, hypoxic respiratory failure and acute respiratory distress syndrome, sepsis and septic shock.

Through creative research in the present application, it is found that chloroquine phosphate has a good protective effect on SARS-CoV-2 infected cells in vitro, with $EC_{50}$=1.13μM, $CC_{50}$>100μM, SI>88.5; the compound of Formula I hydroxychloroquine has a toxicity of only half that of chloroquine phosphate in human body, has a function of inhibiting the replication of SARS-CoV-2, and has a good therapeutic effect in the treatment of diseases caused by SARS-CoV-2.

The present application provides a compound having a structure represented by Formula I or Formula II, a geometric isomer, a pharmaceutically acceptable salt and/or a solvate or a hydrate thereof:

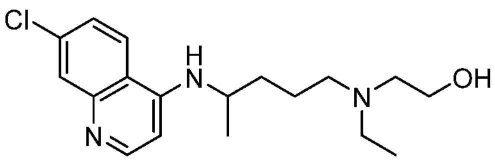

I

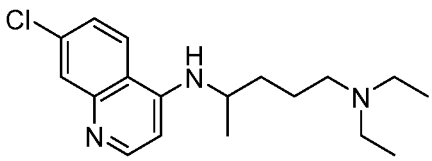

II.

According to the present application, the pharmaceutically acceptable salts of the compound of the present application include an inorganic or organic acid salt thereof and an inorganic or organic base salt thereof. The present application relates to all forms of the above salts, including but not limited to: sodium salt, potassium salt, calcium salt, lithium salt, meglumine salt, hydrochloride salt, hydrobromide salt, hydroiodide salt, nitrate salt, sulfate, hydrogen sulfate, phosphate, hydrogen phosphate, acetate, propionate, butyrate, oxalate, pivalate, adipate, alginate, lactate, citrate, tartrate, succinate, maleate, fumarate, picrate, aspartate, gluconate, benzoate, methanesulfonate, ethanesulfonate, benzenesulfonate, p-toluenesulfonate and embonate and so on.

According to the present application, hydroxychloroquine represented by Formula I or chloroquine represented by Formula II can inhibit virus replication in a cell and reduce viral nucleic acid load in a cell culture.

After creative invention and research, the inventors of the present application have discovered some new features of hydroxychloroquine represented by Formula I or chloroquine represented by Formula II:

hydroxychloroquine represented by Formula I or chloroquine represented by Formula II can reduce the viral nucleic acid load at micromolar concentration level in SARS-CoV-2 infected cells.

The present application also relates to a pharmaceutical composition comprising hydroxychloroquine represented by Formula I and/or chloroquine represented by Formula II, a geometric isomer, a pharmaceutically acceptable salt, a pharmaceutically acceptable solvate or hydrate thereof, and a pharmaceutically acceptable carrier. The pharmaceutical composition can be administered through various routes, such as oral tablet, capsule, powder, oral liquid, injection and transdermal preparation. According to conventional pharmaceutical practices, the pharmaceutically acceptable carrier includes diluent, filler, disintegrant, wetting agent, lubricant, coloring agent, flavoring agent or other conventional additives. The typical pharmaceutically acceptable carrier includes, for example, microcrystalline cellulose, starch, crospovidone, povidone, polyvinylpyrrolidone, maltitol, citric acid, sodium lauryl sulfonate or magnesium stearate, etc.

Another aspect of the present application relates to a pharmaceutical composition, which comprises the compound of the present application and at least one pharmaceutically acceptable carrier. The pharmaceutical composition can be prepared into various forms according to different administration routes.

The present application also relates to use of hydroxychloroquine represented by Formula I or chloroquine represented by Formula II, a geometric isomer, a pharmaceutically acceptable salt, a solvate and/or a hydrate thereof in the manufacture of a medicament for the treatment of a disease or an infection caused by a SARS-CoV-2 (e.g., a

respiratory disease (including but not limited to simple infection (such as fever, cough and sore throat), pneumonia, acute or severe acute respiratory infection, hypoxic respiratory failure and acute respiratory distress syndrome), sepsis and septic shock, etc.),

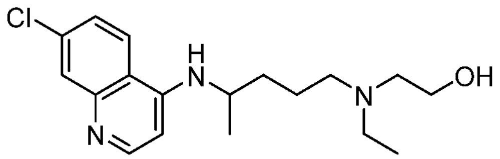

I

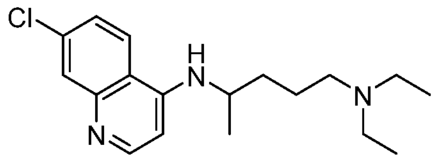

II.

The present application also relates to use of hydroxychloroquine represented by Formula I or chloroquine represented by Formula II, a geometric isomer, a pharmaceutically acceptable salt, a solvate and/or a hydrate thereof in the manufacture of a medicament as a SARS-CoV-2 inhibitor, or in the manufacture of a medicament for inhibiting the replication or reproduction of SARS-CoV-2 in a cell (e.g., a cell of mammal),

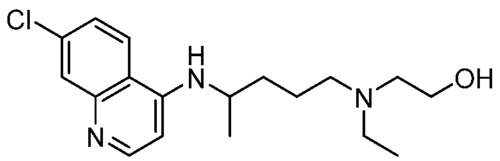

I

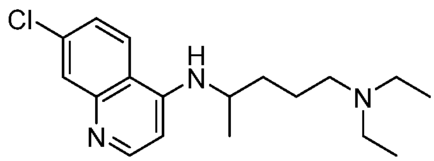

II.

The present application also relates to use of a pharmaceutical composition in the manufacture of a medicament as a SARS-CoV-2 inhibitor, or in the manufacture of a medicament for inhibiting the replication or reproduction of SARS-CoV-2 in a cell (e.g., a cell of mammal), wherein the pharmaceutical composition comprises hydroxychloroquine represented by Formula I or chloroquine represented by Formula II, a geometric isomer, a pharmaceutically acceptable salt, a solvate and/or a hydrate thereof,

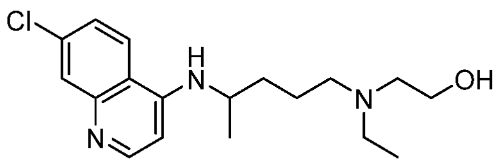

I

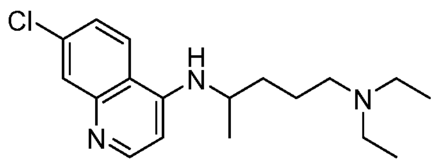

II

preferably, the pharmaceutical composition further comprises a pharmaceutically acceptable carrier or excipient. Specifically, the pharmaceutical composition is a solid preparation, an injection, an external preparation, a spray, a liquid preparation, or a compound preparation.

The present application also relates to use of a pharmaceutical composition in the manufacture of a medicament for the treatment of a disease or an infection caused by a SARS-CoV-2 (e.g., a respiratory disease (including but not limited to simple infection (such as fever, cough and sore throat), pneumonia, acute respiratory infection or severe acute respiratory infection, hypoxic respiratory failure and acute respiratory distress syndrome, sepsis and septic shock, etc.)), wherein the pharmaceutical composition comprises hydroxychloroquine represented by Formula I or chloroquine represented by Formula II, a geometric isomer, a pharmaceutically acceptable salt, a solvate and/or a hydrate thereof,

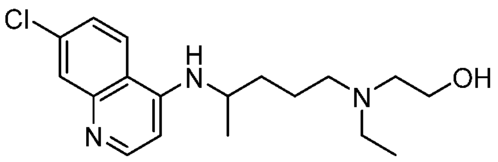
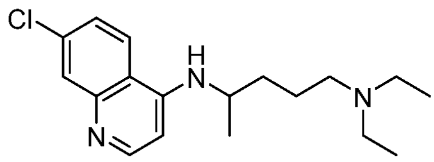

I II

preferably, the pharmaceutical composition further comprises a pharmaceutically acceptable carrier or excipient. Specifically, the pharmaceutical composition is a solid preparation, an injection, an external preparation, a spray, a liquid preparation, or a compound preparation.

The present application also relates to a method for treating and/or preventing a disease or a viral infection in a mammal in need thereof, or a method for inhibiting the replication or reproduction of SARS-CoV-2 in a mammal in need thereof, wherein the method comprises administering to the mammal in need thereof a therapeutically and/or prophylactically effective amount of hydroxychloroquine represented by Formula I or chloroquine represented by Formula II, a geometric isomer, a pharmaceutically acceptable salt, a solvate and/or a hydrates thereof, or a therapeutically and/or prophylactically effective amount of a pharmaceutical composition comprising hydroxychloroquine represented by Formula I or chloroquine represented by Formula II, a geometric isomer, a pharmaceutically acceptable salt, a solvate and/or a hydrate thereof,

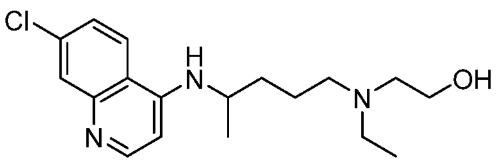
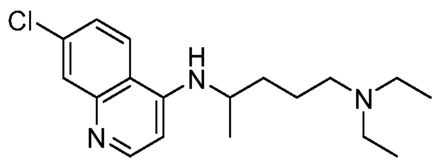

I II

wherein, the disease includes a disease caused by a SARS-CoV-2 (e.g., a respiratory disease (including but not limited to simple infection (such as fever, cough and sore throat), etc., pneumonia, acute respiratory infection or severe acute respiratory infection (SARI), hypoxic respiratory failure and acute respiratory distress syndrome, sepsis and septic shock, etc.)), the viral infection includes an infection caused by a SARS-CoV-2.

The present application also relates to hydroxychloroquine represented by Formula I or chloroquine represented by Formula II, a geometric isomer, a pharmaceutically acceptable salt, a solvate and/or a hydrate thereof, or a pharmaceutical composition comprising hydroxychloroquine represented by Formula I or chloroquine represented by Formula II, a geometric isomer, a pharmaceutically acceptable salt, a solvate and/or a hydrate thereof, for use as a SARS-CoV-2 inhibitor, or for use in inhibiting the replication or reproduction of SARS-CoV-2 in a cell (e.g., a cell in mammal),

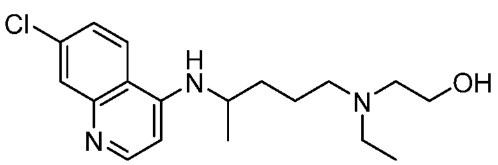
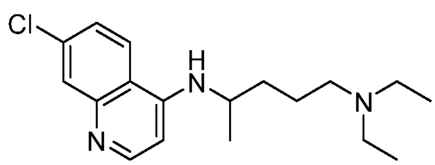

I II

preferably, the pharmaceutical composition also comprises a pharmaceutically acceptable carrier or excipient.

The present application also relates to hydroxychloroquine represented by Formula I or chloroquine represented by Formula II, a geometric isomer, a pharmaceutically acceptable salt, a solvate and/or hydrate thereof, or a pharmaceutical composition comprising hydroxychloroquine represented by Formula I or chloroquine represented by Formula II, a geometric isomer, a pharmaceutically acceptable salt, a solvate and/or a hydrate thereof, for use in the treatment of a disease or an infection caused by a SARS-CoV-2 (e.g., a respiratory disease (including but not limited to simple infection (such as fever, cough and sore throat), pneumonia, acute respiratory infection or severe acute respiratory infection, hypoxic respiratory failure and acute respiratory distress syndrome), sepsis and septic shock, etc.),

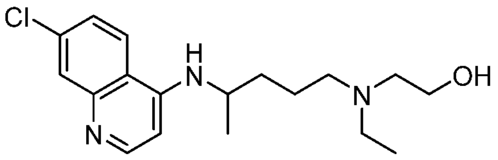

I

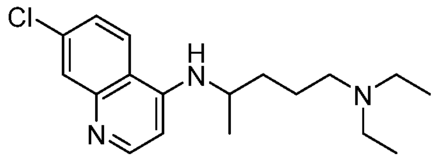

II

preferably, the pharmaceutical composition further comprises a pharmaceutically acceptable carrier or excipient.

In some embodiments, the pharmaceutically acceptable salt of hydroxychloroquine represented by Formula I or chloroquine represented by Formula II includes an inorganic or organic acid salt thereof and an inorganic or organic base salt thereof,

the pharmaceutically acceptable salt includes, but is not limited to: sodium salt, potassium salt, calcium salt, lithium salt, meglumine salt, hydrochloride salt, hydrobromide salt, hydroiodide salt, nitrate salt, sulfate, hydrogen sulfate, phosphate, hydrogen phosphate, acetate, propionate, butyrate, oxalate, pivalate, adipate, alginate, lactate, citrate, tartrate, succinate, maleate, fumarate, picrate, aspartate, gluconate, benzoate, methanesulfonate, ethanesulfonate, benzenesulfonate, p-toluenesulfonate and embonate and so on.

In some embodiments, the pharmaceutically acceptable salt of chloroquine of Formula II is chloroquine sulfate.

In some embodiments, the pharmaceutically acceptable salt of chloroquine of Formula II is chloroquine phosphate.

In some embodiments, the disease caused by a SARS-CoV-2 described in the present application is COVID-19.

In the present application, the official name of the term "2019 novel coronavirus (2019-nCoV)" is severe acute respiratory syndrome coronavirus 2 (SARS-CoV-2).

In the present application, the official name of the term "disease caused by 2019 novel coronavirus (2019-nCoV)" is COVID-19.

The pharmaceutical composition described in the present application can be administered through various routes, such as oral tablet, capsule, powder, oral liquid, injection and transdermal preparation. According to conventional pharmaceutical practices, the pharmaceutically acceptable carrier includes diluent, filler, disintegrant, wetting agent, lubricant, coloring agent, flavoring agent or other conventional additives. The typical pharmaceutically acceptable carrier includes, for example, microcrystalline cellulose, starch, crospovidone, povidone, polyvinylpyrrolidone, maltitol, citric acid, sodium lauryl sulfonate or magnesium stearate, etc.

In some embodiments, the mammal includes bovine, equine, caprid, suidae, canine, feline, rodent, primate, wherein the preferred mammal is a human.

The pharmaceutical composition described in the present application can be prepared into various forms according to different administration routes.

According to the present application, the pharmaceutical composition can be administered in any one of the following routes: oral administration, spray inhalation, rectal administration, nasal administration, buccal administration, vaginal administration, topical administration, parenteral administration such as subcutaneous, intravenous, intramuscular, intraperitoneal, intrathecal, intraventricular, intrasternal and intracranial injection or infusion, or administration with the help of an explant reservoir. Among them, oral, intraperitoneal or intravenous administration is preferred.

When orally administered, the chloroquine or hydroxychloroquine, a geometric isomer, a pharmaceutically acceptable salt, a solvate and/or a hydrate thereof can be prepared into any form of orally acceptable preparation, including but not limited to a tablet, a capsule, an aqueous solution or an aqueous suspension. Generally, the carrier for use in a tablet includes lactose and corn starch, and a lubricant such as magnesium stearate can also be added. The diluent for use in a capsule generally includes lactose and dry corn starch. The aqueous suspension is usually used by mixing an active ingredient with a suitable emulsifier and a suitable suspending agent. If necessary, a sweetener, a flavoring agent or a coloring agent can also be added to the above-mentioned forms of oral preparation.

When rectally administered, the chloroquine or hydroxychloroquine, a geometric isomer, a pharmaceutically acceptable salt, a solvate, and/or a hydrate thereof can generally be prepared in a form of suppository, which is prepared by mixing the drug with a suitable non-irritating excipient. The excipient is present in solid state at room temperature, but melts at the rectal temperature to release the drug. Such excipient includes cocoa butter, beeswax and polyethylene glycol.

When topically administered, especially for the treatment of easily accessible affected-surface or organ, such as eye, skin, or lower intestinal neurological disease by topical application, the chloroquine or hydroxychloroquine, a geometric isomer, a pharmaceutically acceptable salt, a solvate and/or a hydrate thereof can be prepared in various forms of topical preparations according to different affected-surfaces or organs, the specific instructions are as follows:

When topically administered to eye, the chloroquine or hydroxychloroquine, a geometric isomer, a pharmaceutically

acceptable salt, a solvate and/or a hydrate thereof can be formulated into a preparation form such as micronized suspension or solution, the carrier used is isotonic sterile saline with a certain pH, and a preservative such as benzyl chloride alkoxide may or may not be added. In addition, for administration to eye, the compound can also be prepared in a form of ointment such as vaseline ointment.

When topically administered to skin, the chloroquine or hydroxychloroquine, a geometric isomer, a pharmaceutically acceptable salts, a solvate and/or a hydrate thereof can be prepared into a suitable form such as an ointment, a lotion or a cream, in which the active ingredient is suspended or dissolved in one or more carriers. The carrier for use in an ointment includes, but is not limited to mineral oil, liquid petrolatum, white petrolatum, propylene glycol, polyethylene oxide, polypropylene oxide, emulsifying wax, and water. The carrier for use in a lotion or a cream includes, but is not limited to mineral oil, sorbitan monostearate, Tween-60, cetyl ester wax, hexadecenyl aryl alcohol, 2-octyldodecanol, benzyl alcohol and water.

When topically administered to lower intestinal tract, the chloroquine or hydroxychloroquine, a geometric isomer, a pharmaceutically acceptable salt, a solvate and/or a hydrate thereof can be prepared into a form such as rectal suppository as described above or a suitable enema preparation form, in addition, a topical transdermal patch can also be used.

The chloroquine or hydroxychloroquine, a geometric isomer, a pharmaceutically acceptable salt, a solvate and/or a hydrate thereof can also be administered in a preparation form of sterile injection, including sterile injectable aqueous solution or oil suspension, or sterile injectable solutions, wherein the usable carrier and solvent includes water, Ringer's solution and isotonic sodium chloride solution. In addition, a sterilized non-volatile oil such as monoglyceride or diglyceride can also be used as solvent or suspension media.

The drugs of the above various preparation forms can be prepared according to conventional methods in the pharmaceutical field.

In the present application, the term "therapeutically effective amount" or "prophylactically effective amount" refers to an amount that is sufficient to treat or prevent a patient's disease but is sufficiently low to avoid serious side effects (at a reasonable benefit/risk ratio) within a reasonable medical judgment. The therapeutically effective amount of the compound will change according to the factors such as the selected specific compound (e.g., considering the efficacy, effectiveness, and half-life of compound), the selected administration route, the treated disease, the severity of the treated disease, the patient's age, size, weight and physical disease, medical history, duration of treatment, nature of concurrent therapy, desired therapeutic effect, etc., but can still be routinely determined by those skilled in the art.

In addition, it should be noted that the specific dosage and method of using the chloroquine or hydroxychloroquine, a geometric isomer, a pharmaceutically acceptable salt, a solvate and/or a hydrate thereof for different patients depends on many factors, including the patient's age, weight, gender, natural health status, nutritional status, active strength of drug, administration time, metabolic rate, severity of disease, and subjective judgment of physician. Herein it is preferred to use a dosage between 0.001-100 mg/kg body weight/day.

## Brief Description of the Drawings

Figure 1 shows the experimental results of effectiveness and safety of chloroquine phosphate on vero-E6 cells infected by SARS-CoV-2, wherein (A) shows the half-maximal effective concentration ($\mu$M) and the half-cytotoxic concentration ($\mu$M) of chloroquine phosphate 48 hours after the cells were infected with SARS-CoV-2; (B) shows the immunofluorescence image after the cells infected with SARS-CoV-2 were treated with chloroquine phosphate.

Figure 2 shows the research results of effectiveness and safety of chloroquine phosphate and hydroxychloroquine against SARS-CoV-2 in vitro (vero-E6 cells), wherein (A) shows the cytotoxicities and the half-cytotoxic concentrations of chloroquine phosphate and hydroxychloroquine; (B) shows the antiviral activities and half-maximal effective concentrations of chloroquine phosphate and hydroxychloroquine at MOI of 0.01; (C) shows the antiviral activities and half-maximal effective concentrations of chloroquine phosphate and hydroxychloroquine at MOI of 0.02; (D) shows the antiviral activities and half-maximal effective concentrations of chloroquine phosphate and hydroxychloroquine at MOI of 0.2; (E) shows the antiviral activities and half-maximal effective concentrations of chloroquine phosphate and hydroxychloroquine at MOI of 0.8.

Figure 3 shows the research results of antiviral mechanism of chloroquine phosphate and hydroxychloroquine, wherein (A) shows the quantitative analysis results of co-localization of virus particles and early endosomes or lysosomes in cells after treatment with chloroquine phosphate and hydroxychloroquine; (B) shows the confocal micrograph of representative co-localization.

**Specific Models for Carrying Out the Invention**

The technical solutions in the examples of the present application will be described clearly and completely in combination with the drawings in the examples of the present application. Obviously, the described examples are only a part of the examples of the present application, rather than all the examples. The following description of at least one exemplary example is actually only illustrative, and is not intended to limit the present application and its application or use. On the basis of the examples in the present application, all other examples obtained by those skilled in the art without creative work shall fall within the protection scope of the present application.

When the specific techniques or conditions are not indicated in the Examples, the Examples are carried out according to the techniques or conditions described in the literature in the field or according to the product specifications. The materials or equipment used herein, the manufacturers of which are not indicated, are the conventional products that are commercially available.

Example 1: Experiment on chloroquine phosphate reducing viral nucleic acid load in SARS-CoV-2 infected cells

(1) Drug treatment of virus-infected cells

Vero E6 cells (purchased from ATCC, Catalog No. 1586) was inoculated on a 24-well plate, cultured for 24 hours; then virus infection was carried out. Specifically, SARS-CoV-2 (2019-nCoV) virus (nCoV-2019BetaCoV/Wuhan/WIV04/2019 strain, preserved by Wuhan Institute of Virology, Chinese Academy of Sciences) was diluted with 2% cell maintenance solution (formulation: FBS (purchased from Gibco company, Catalog No. 16000044) was added to MEM (purchased from Gibco, Catalog No. 10370021) at a volume ratio of 2%, thereby obtaining the 2% cell maintenance solution) to a corresponding concentration, and then added to a 24-well plate so that each well contained a viral load of 100$TCID_{50}$. Next, chloroquine phosphate and hydroxychloroquine (chloroquine phosphate was purchased from Sigma-Aldrich, Catalog No. C6628; hydroxychloroquine was purchased from MCE company, Catalog No. HY-B1370) were diluted with 2% cell maintenance solution to corresponding concentrations and added separately to the corresponding wells, so that the final concentrations of the drugs were 50μM, 16.67μM, 5.56μM, 1.85μM, 0.62μM, 0.21μM, 0.068μM, respectively, then the plate was placed in a 37°C, 5% $CO_2$ incubator and cultured for 48 hours. To the vehicle control group, the 2% cell maintenance solution without any test drugs was added.

(2) RNA extraction

RNA extraction kit was purchased from Qiagen Company, Catalog No. 74106. The consumables (spin columns, RNase-free 2ml collection tubes, etc.) and reagents (RLT, RW1, RPE, RNase-free water, etc.) involved in the following RNA extraction steps were part of the kit. The following extraction steps were recommended steps in the kit instruction.

1) 100μL of the supernatant was taken from the tested plate and added to a nuclease-free EP tube (purchased from Axygen, Catalog No. mct-150-c), then 350μL of Buffer RLT was added to each well and mixed by beating with a transfer liquid gun until complete lysis was achieved, then centrifugation was carried out to obtain a supernatant;

2) an equal volume of 70% ethanol was added to the supernatant obtained in 1) and mixed well;

3) the mixture solution obtained in 2) was transferred to a RNase-free spin column, and centrifuged at 12000 rpm for 15 seconds, and the waste liquid was discarded;

4) 700μL of Buffer RW1 was added to the spin column, and centrifuged at 12000 rpm for 15 seconds to clean the spin column, and the waste liquid was discarded;

5) 500μL of Buffer RPE was added to the spin column, and centrifuged at 12000 rpm for 15 seconds to clean the spin column, and the waste liquid was discarded;

6) 500μL of Buffer RPE was added to the spin column, and centrifuged at 12000 rpm for 2min to clean the spin column, the waste was discarded, and then the spin column was transferred to a new RNase-free 2ml collection tube of step 7);

7) a new RNase-free 2ml collection tube was used for replacement, centrifugation was carried out at 12000 rpm for 1 min, the spin column was dried, and then the spin column was transferred to a 1.5ml collection tube in step 8);

8) a new 1.5ml collection tube was used for replacement, in which the spin column dried in step 7) was placed, and 30μl of RNase-free water was added to the spin column, and centrifugation was carried out at 12000 rpm for 2 minutes, the obtained eluate contained the corresponding RNA, then the RNase inhibitor (purchased from NEB company, Catalog No. M0314L) was added, and Nano Drop (purchased from Thermo scientific, Nano Drop One) was used to detect each RNA concentration.

(3) RNA reverse transcription

In the experiment, the reverse transcription kit (PrimeScript™ RT reagent Kit with gDNA Eraser, Catalog No. RR047Q) produced by TaKaRa was used for RNA reverse transcription. The steps were as follows.

① Removal of gDNA: RNA samples of each experimental group were collected, 1 μg of each sample was taken for reverse transcription. First, 2μl of 5× gDNA Eraser Buffer was added to the RNA sample of each experimental group, the reaction system was supplemented with RNase-free water to reach 10μl, mixed well, and subjected to water bath at 42°C for 2 min to remove the gDNA that might be present in the sample;

② Reverse transcription: Appropriate amounts of enzyme, primer Mix and reaction buffer were added to the sample obtained in ①, RNase-free water was added to supplement to reach a volume of 20 μl, the reaction was performed in a water bath at 37°C for 15 minutes, and then in water bath at 85°C for 5 seconds, to obtain cDNA by transcription.

(4) Real-time PCR

Fluorescence quantitative PCR was used to detect the number of copies per milliliter of the original virus solution.

The reaction system was mixed by using TB Green Premix (Takara, Cat#RR820A), and the amplification reaction and reading were carried out with StepOne Plus Real-time PCR instrument (brand: ABI). The copy number contained in per milliliter of the original virus solution was calculated. The steps were as follows:

① Establishment of standard product: the plasmid pMT-RBD (the plasmid was preserved by Wuhan Institute of Virology, Chinese Academy of Sciences) was diluted to $5\times10^8$ copies/μL, $5\times10^7$ copies/μL, $5\times10^6$ copies/μL, $5\times10^5$ copies/ μL, $5\times10^4$ copies/μL, $5\times10^3$ copies/μL, $5\times10^2$ copies/μL, respectively. 2 μL of standard product or cDNA template was taken for qPCR reaction.

② The primer sequences used in the experiment were as follows (all indicated in the 5'-3' direction):

RBD-qF: CAATGGTTTAACAGGCACAGG

RBD-qR: CTCAAGTGTCTGTGGATCACG

③ The reaction procedure was as follows:

Pre-denaturation: 95°C for 5 minutes;

Cycle parameters: 95°C for 15 seconds, 54°C for 15 seconds, 72°C for 30 seconds, a total of 40 cycles.

(5) Cytotoxicity test of drugs

The cytotoxicity test of drugs was carried out by using CCK-8 kit (Beyotime). Specific steps were as follows:

① $1\times10^4$ Vero-E6 cells were inoculated in a 96-well plate and incubated at 37°C for 8 hours.
② The drug was diluted with DMSO to an appropriate concentration of mother solution, and then diluted with MEM (purchased from Gibco, Catalog No. 10370021) medium containing 2% FBS (purchased from Gibco company, Catalog No. 16000044) to the same concentration as that for the drug treatment. The original medium in the 96-well plate was discarded, 100 μL of the drug-containing MEM medium was taken and added to the cells, and three replicate wells were set for each concentration. A vehicle control (adding DMSO and medium to cells in wells, without adding drug) and a blank control (adding DMSO and medium to the wells, without cells) were set up. After the drug was added, the cells were incubated at 37°C for 48 hours.
③ 20 μL of CCK-8 solution (Beyotime) was added to the well to be tested, mixed gently without generating bubbles, and incubated subsequently at 37°C for 2 hours. $OD_{450}$ was read on a microplate reader (purchased from Molecular

Devices, model: SpectraMax M5), and the reading was substituted into the following formula to calculate cell viability:

$$\text{Cell viability (\%)} = (A_{\text{(drug treatment group)}} - A_{\text{(blank control)}}) / (A_{\text{(vehicle control)}} - A_{\text{(blank control)}}) \times 100\%$$

Wherein, A was the reading of the microplate reader.

(6) Experimental results

The results of the virus proliferation inhibition experiment showed that the test compound chloroquine phosphate at concentrations of 50μM, 16.67μM, and 5.56μM could effectively inhibit the replication of SARS-CoV-2 virus genome in the infected supernatant.

The results of cytotoxicity test (see: Figure 1) showed that the treatment of the test compound chloroquine phosphate did not change cell viability at all test concentrations, that was, the test compound had no toxic effect on cells at all concentrations.

According to calculations, the half-maximal effective concentration ($EC_{50}$) of chloroquine phosphate was 1.13 μM, the half-cytotoxic concentration ($CC_{50}$) to cells was greater than 100 μM, and the selectivity index (SI) was greater than 88.5. The results showed that chloroquine phosphate could effectively block SARS-CoV-2 virus infection at low micromolar concentrations, and showed a higher SI.

Example 2: Experiment on chloroquine phosphate and hydroxychloroquine reducing viral nucleic acid load in SARS-CoV-2 infected cells at 4 multiplicities of infections (MOIs)

(1) Drug treatment

Vero E6 cells were inoculated into a 24-well plate, cultured for 24 hours, and then subjected to virus infection. Four groups of different infection doses were set, which are MOI of 0.01, MOI of 0.02, MOI of 0.2 and MOI of 0.8, respectively. SARS-CoV-2 (2019-nCoV) viruses were diluted with 2% cell maintenance solution to the corresponding concentration, and then added to a 24-well plate so that the cell viral load in each well reached the set infection dose. Then, chloroquine phosphate and hydroxychloroquine were separately diluted with 2% cell maintenance solution to reach corresponding concentrations, and added to the corresponding wells so that the final concentrations of the drugs were 50μM, 16.67μM, 5.56μM, 1.85μM, 0.62μM, 0.21μM, 0.068μM, respectively, then they were placed in a 37°C, 5% $CO_2$ incubator and cultured for 48 hours. To the cell control group, 2% cell maintenance solution without any test drugs was added.

(2) RNA extraction

RNA extraction was carried out by referring to the method as described in step (2) in Example 1.

(3) RNA reverse transcription

RNA reverse transcription was carried out by referring to the method as described in step (3) in Example 1.

(4) Real-time PCR

With reference to the method as described in step (4) in Example 1, fluorescence quantitative PCR was used to detect the number of copies per milliliter of the original virus solution.

(5) Cytotoxicity test of drugs

Cytotoxicity test of drugs to cells was carried out by referring to the method as described in step (5) in Example 1.

(6) Experimental results

The results of cytotoxicity test (see: A in Figure 2) showed that the treatment of the test compounds did not change the cell viability at the test concentration of 100 μM, that was, the test compounds had no toxic effect on the cells at this concentration. According to calculations, the $CC_{50}$ of hydroxychloroquine was greater than 249.50 μM, and the $CC_{50}$ of chloroquine phosphate was greater than 273.20 μM.

The results of the virus proliferation inhibition experiment showed that the test compounds can effectively inhibit the replication of SARS-CoV-2 viral genome in the infection supernatant at the MOI of 0.01, 0.02, 0.2 and 0.8 (see: B to E in Figure 2). At different MOIs, the ECso values of the test compounds were as follows:
MOI=0.01

Hydroxychloroquine: ECso=4.51 μM; Chloroquine phosphate: ECso=2.71 μM;

MOI=0.02

Hydroxychloroquine: $EC_{50}$=4.06 μM; Chloroquine phosphate: $EC_{50}$=3.81 μM;

MOI=0.2

Hydroxychloroquine: $EC_{50}$=17.31 μM; Chloroquine phosphate: $EC_{50}$=7.14 μM;

MOI=0.8

Hydroxychloroquine: $EC_{50}$=12.96 μM; Chloroquine phosphate: $EC_{50}$=7.36 μM.

Example 3: Experiment on mechanism of chloroquine phosphate and hydroxychloroquine inhibiting SARS-CoV-2 virus invasion

(1) Experimental method

1) chloroquine phosphate or hydroxychloroquine was formulated with MEM medium containing 2% FBS to prepare 50 μM of drug-containing culture solution, and then veroE6 cells were treated with the above-mentioned drug-containing culture solution for 1 hour;

2) the veroE6 cells were allowed to bind with SARS-CoV-2 virus for 1h at 4°C (at MOI of 10);

3) the veroE6 cells were washed twice with PBS (purchased from Gibco, Catalog No. C10010500BT) to remove unbound virus particles, freshly pre-warmed MEM culture medium containing 2% FBS was added, and then the veroE6 cells were incubated at 37°C for 90 min;

4) the cells were fixed, and the immunofluorescence staining of cells was performed by using anti-virus NP protein antibody (red) (preserved by Wuhan Institute of Virology, Chinese Academy of Sciences) and anti-early endosomal protein EEA antibody (green) (purchased from Cell Signaling Technology, Catalog No. 48453) or anti-lysosomal protein LAMP antibody (green) (purchased from Cell Signaling Technology company, Catalog No. 3243); the nuclei were stained with Hoechst (blue) (purchased from Invitrogen company, Catalog No. H21492);

5) quantitative analysis of co-localization of virus particles and endosomes of the cells in each group was performed, and co-localization rate was calculated.

(2) Experimental results

The results of immunofluorescence analysis (IFA) and confocal micrograph analysis (see: Figure 3) showed that after treatment with chloroquine phosphate or hydroxychloroquine, the co-localization rate of virus particles and early endosomes (EES) increased significantly (the co-localization rate of chloroquine phosphate was 35.3%; the co-localization rate of hydroxychloroquine was 29.2%; P<0.001), and the co-localization rate of virus particles and endolysosomes (ELs) was significantly reduced (the co-localization rate of chloroquine phosphate was 2.4%; the co-localization rate of hydroxychloroquine was 0.03%; P<0.001), indicating that chloroquine phosphate or hydroxychloroquine inhibited viral infection by blocking the transport process of SARS-CoV-2 virus from early endosome to lysosome.
Finally, it should be noted that the above examples are only used to illustrate the technical solutions of the present application rather than to limit them; although the present application has been described in detail with reference

to the preferred examples, those of ordinary skill in the art should understand that the specific implementation of the present application may be modified or some technical features may be equivalently replaced without departing from the spirit of the technical solutions of the present application, and all of them shall be covered by the scope of the technical solution sought to be protected by the present application.

## Claims

1. Use of hydroxychloroquine represented by Formula I or chloroquine represented by Formula II, a geometric isomer, a pharmaceutically acceptable salt, a solvate and/or a hydrate thereof in the manufacture of a medicament for the treatment of a disease or an infection caused by a SARS-CoV-2 (e.g., a respiratory disease (including but not limited to simple infection (such as fever, cough and sore throat), pneumonia, acute respiratory infection or severe acute respiratory infection, hypoxic respiratory failure and acute respiratory distress syndrome, sepsis and septic shock, etc.)),

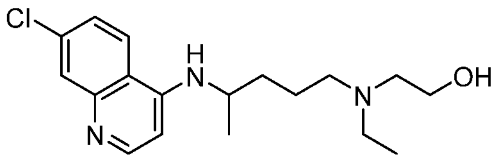

I

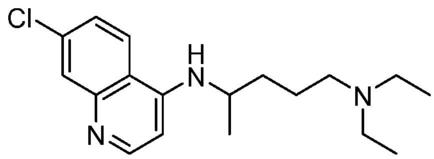

II.

2. Use of hydroxychloroquine represented by Formula I or chloroquine represented by Formula II, a geometric isomer, a pharmaceutically acceptable salt, a solvate and/or a hydrate thereof in the manufacture of a medicament as a SARS-CoV-2 inhibitor, or in the manufacture of a medicament for inhibiting the replication or reproduction of SARS-CoV-2 in a cell (e.g., a cell of mammal),

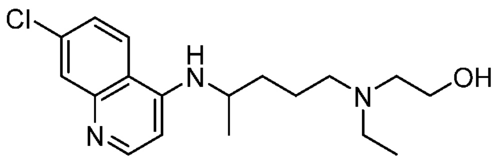

I

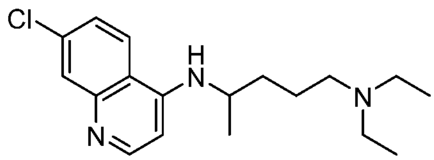

II.

3. Use of a pharmaceutical composition in the manufacture of a medicament as SARS-CoV-2 inhibitor, or in the manufacture of a medicament for inhibiting the replication or reproduction of SARS-CoV-2 in a cell (e.g., a cell of mammal),
wherein the pharmaceutical composition comprises hydroxychloroquine represented by Formula I or chloroquine represented by Formula II, a geometric isomer, a pharmaceutically acceptable salt, a solvate and/or a hydrate thereof,

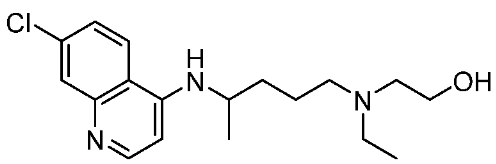

I

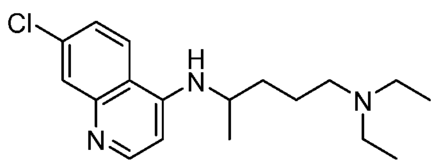

II

preferably, the pharmaceutical composition further comprises a pharmaceutically acceptable carrier or excipient; specifically, the pharmaceutical composition is a solid preparation, an injection, an external preparation, a spray, a liquid preparation, or a compound preparation.

4. Use of a pharmaceutical composition in the manufacture of a medicament for the treatment of a disease or an

infection caused by a SARS-CoV-2 (e.g., a respiratory disease (including but not limited to simple infection (such as fever, cough and sore throat), pneumonia, acute respiratory infection or severe acute respiratory infection, hypoxic respiratory failure and acute respiratory distress syndrome), sepsis and septic shock, etc.), wherein the pharmaceutical composition comprises hydroxychloroquine represented by Formula I or chloroquine represented by Formula II, a geometric isomer, a pharmaceutically acceptable salt, a solvate and/or a hydrate thereof,

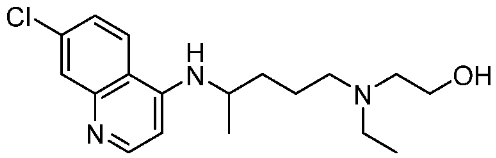

I

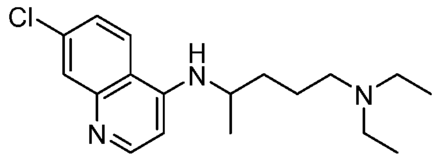

II

preferably, the pharmaceutical composition further comprises a pharmaceutically acceptable carrier or excipient; specifically, the pharmaceutical composition is a solid preparation, an injection, an external preparation, a spray, a liquid preparation, or a compound preparation.

**5.** A method for treating and/or preventing a disease or a viral infection in a mammal in need thereof, or a method for inhibiting the replication or reproduction of SARS-CoV-2 in a mammal in need, wherein the method comprises administering to the mammal in need thereof a therapeutically and/or prophylactically effective amount of hydroxychloroquine represented by Formula I or chloroquine represented by Formula II, a geometric isomer, a pharmaceutically acceptable salt, a solvate and/or a hydrates thereof, or a therapeutically and/or prophylactically effective amount of a pharmaceutical composition comprising hydroxychloroquine represented by Formula I or chloroquine represented by Formula II, a geometric isomer, a pharmaceutically acceptable salt, a solvate and/or a hydrate thereof,

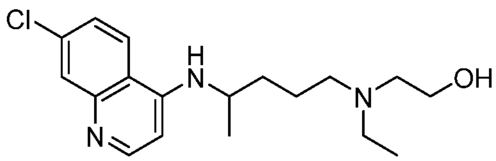

I

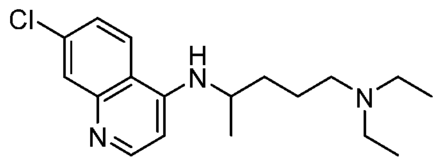

II

wherein, the disease includes a disease caused by a SARS-CoV-2 (e.g., a respiratory disease (including but not limited to simple infection (such as fever, cough and sore throat), pneumonia, acute respiratory infection or severe acute respiratory infection (SARI), hypoxic respiratory failure and acute respiratory distress syndrome), sepsis and septic shock, etc.), the viral infection includes an infection caused by a SARS-CoV-2.

**6.** Hydroxychloroquine represented by Formula I or chloroquine represented by Formula II, a geometric isomer, a pharmaceutically acceptable salt, a solvate and/or a hydrate thereof, or a pharmaceutical composition comprising hydroxychloroquine represented by Formula I or chloroquine represented by Formula II, a geometric isomer, a pharmaceutically acceptable salt, a solvate and/or a hydrate thereof, for use as a SARS-CoV-2 inhibitor, or for use in inhibiting the replication or reproduction of SARS-CoV-2 in a cell (e.g., a cell of mammal),

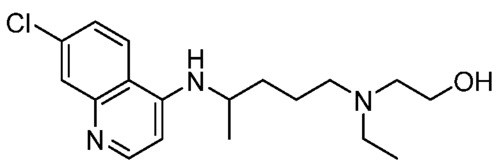

I

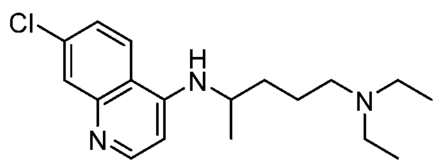

II

preferably, wherein the pharmaceutical composition further comprises a pharmaceutically acceptable carrier or excipient.

7. Hydroxychloroquine represented by Formula I or chloroquine represented by Formula II, a geometric isomer, a pharmaceutically acceptable salt, a solvate and/or a hydrate thereof, or a pharmaceutical composition comprising hydroxychloroquine represented by Formula I or chloroquine represented by Formula II, a geometric isomer, a pharmaceutically acceptable salt, a solvate and/or hydrate thereof, for use in treating a disease or an infection caused by a SARS-CoV-2 (e.g., a respiratory disease (including but not limited to simple infection (such as fever, cough and sore throat), pneumonia, acute respiratory infection or severe acute respiratory infection, hypoxic respiratory failure and acute respiratory distress syndrome), sepsis and septic shock, etc.),

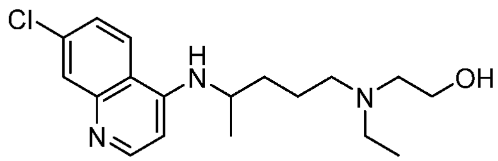

I

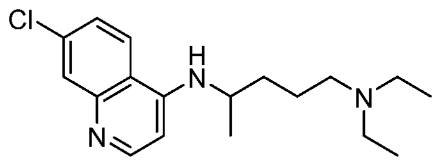

II

preferably, wherein the pharmaceutical composition further comprises a pharmaceutically acceptable carrier or excipient.

8. The use according to any one of claims 1 to 4, the method according to claim 5 or the compound or composition according to claim 6 or 7, wherein the pharmaceutically acceptable salt of hydroxychloroquine represented by Formula I or chloroquine represented by Formula II includes an inorganic or organic acid salt thereof, and an inorganic or organic base salt thereof,

for example, the pharmaceutically acceptable salt includes, but is not limited to: sodium salt, potassium salt, calcium salt, lithium salt, meglumine salt, hydrochloride salt, hydrobromide salt, hydroiodide salt, nitrate salt, sulfate, hydrogen sulfate, phosphate, hydrogen phosphate, acetate, propionate, butyrate, oxalate, pivalate, adipate, alginate, lactate, citrate, tartrate, succinate, maleate, fumarate, picrate, aspartate, gluconate, benzoate, methanesulfonate, ethanesulfonate, benzenesulfonate, p-toluenesulfonate and embonate and so on, for example, chloroquine sulfate, for example, chloroquine phosphate.

9. The use according to any one of claims 1 to 4, the method according to claim 5 or the compound or composition according to claim 6 or 7, wherein the disease caused by SARS-CoV-2 is COVID-19.

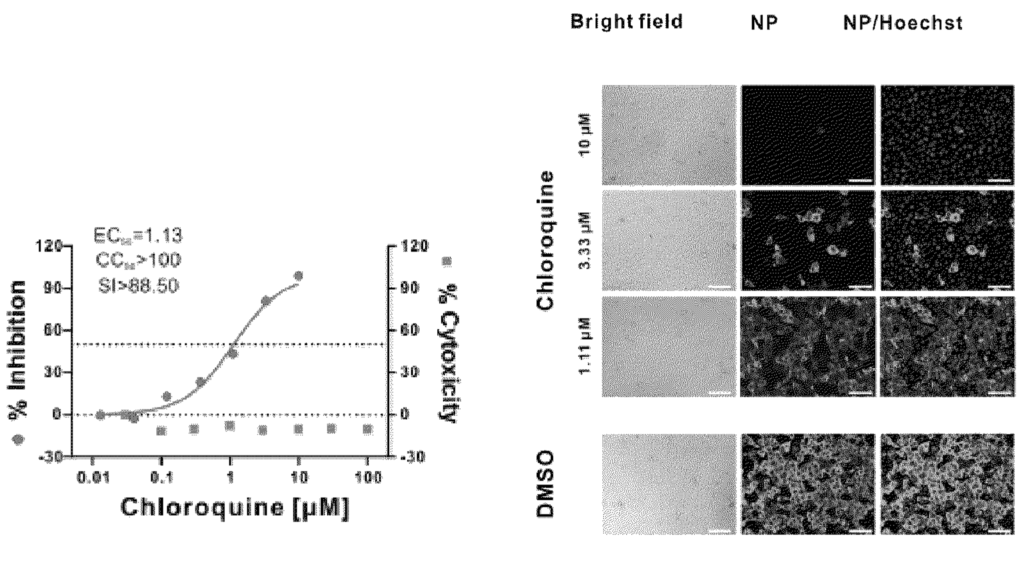


A B

Figure 1

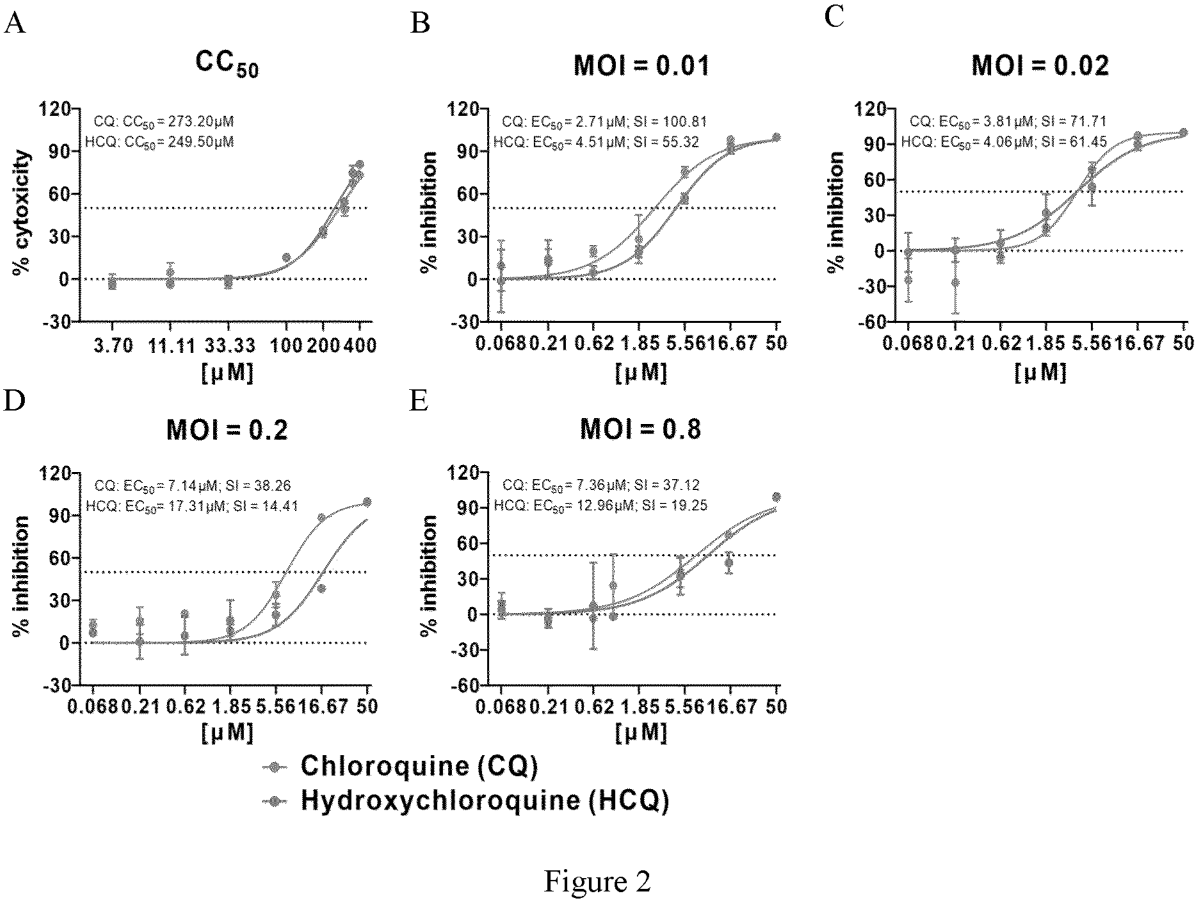


Figure 2

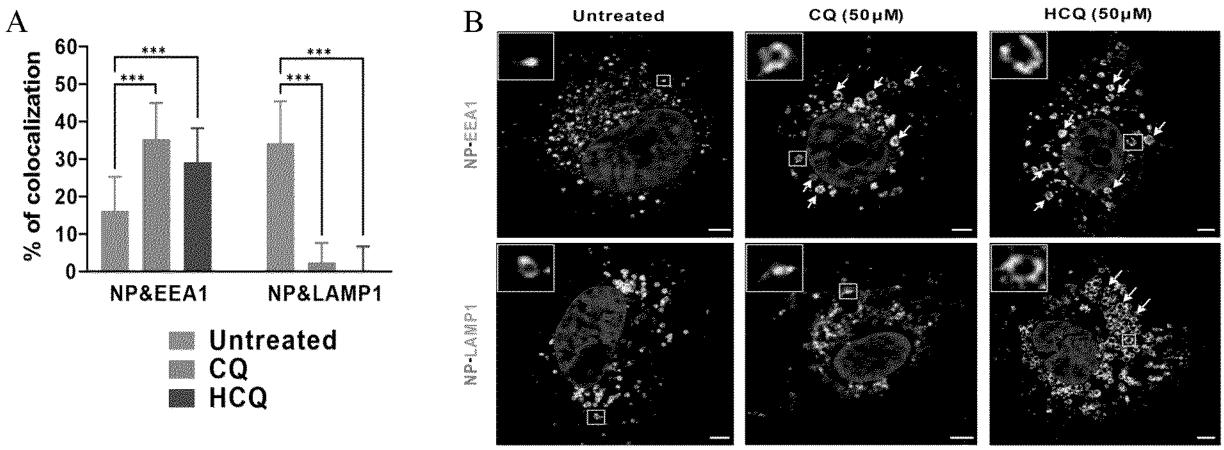
A
60
50
40
30
20
10
0
% of colocalization
***
***
***
***
NP&EEA1
NP&LAMP1
Untreated
CQ
HCQ
B
Untreated
CQ (50μM)
HCQ (50μM)
NP-EEA1
NP-LAMP1

Figure 3

## INTERNATIONAL SEARCH REPORT

International application No.
**PCT/CN2020/093989**

**A. CLASSIFICATION OF SUBJECT MATTER**

A61K 31/4706(2006.01)i; A61K 31/5415(2006.01)i; A61P 31/14(2006.01)i; A61P 11/00(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS, CNTXT, SIPOABS, DWPI, WOXTX, EPTXT, USTXT, CNKI, 万方, WANFANG, 读秀, DUXIU, PUBMED, ISI_Web of Science, Science Direct, STNext: 中国人民解放军军事科学院军事医学研究院, 钟武, 肖庚富, 胡志红, 王曼丽, 张磊, 曹瑞源, 李薇, 樊士勇, 周辛波, 李松, 氯喹, 氯喹啉, 氯化喹啉, 氯奎, 羟基氯喹, 羟氯奎, 二磷酸羟氯喹, 羟氯喹硫酸盐, 硫酸羟基氯喹, 硫酸羟氯喹, 硫酸氯喹, 氯喹啉二磷酸盐, 二磷酸氯喹, 磷酸氯奎, 氯喹磷酸盐, 磷酸氯哇, 氯喹二磷酸盐, 新冠肺炎, 新型冠状病毒肺炎, 冠状病毒, 新冠, Chloroquine, Chlorochin, Aralen, Nivaquine, Chingaminum, Delagil, SN-7618, RP-3377, Aralen, Artrichin, Bemaphate, Capquin, Nivaquine B, Rsoquine, Reumachlor, Sanoquin, CQ, Oxychloroquine, Hydroxychloroquine, Plaquenil, Ercoquin, Quensyl, aminoquinoline, HCQ, HQ, Plaquenil.TM., ChQ, hydroxy-chloroquine, Ercoquin, Plaquenil Sulfate, Quensyl, HCQS, Arechin, Avloclor, Imagon, Malaquin, Resochin, Tresochin, chloroquini phosphas, Chloroquine Phosphate, SARS, covid-19, SARS-CoV-2, severe acute respiratory syndrome, corona virus 2, 2019-nCoV, coronavirus disease 2019, CAS: 54-05-7, 6168-85-0, 747-36-4, 50-63-5

**C. DOCUMENTS CONSIDERED TO BE RELEVANT**

| Category* | Citation of document, with indication, where appropriate, of the relevant passages | Relevant to claim No. |
|---|---|---|
| E | CN 111658648 A (INSTITUTE OF MILITARY MEDICINE, ACADEMY OF MILITARY SCIENCES OF PLA) 15 September 2020 (2020-09-15)<br>claims 1-9 | 1-9 |
| PX | GUO, Deying. "Old Weapon for New Enemy: Drug Repurposing for Treatment of Newly Emerging Viral Diseases,"<br>*Virologica Sinica,* Vol. 35, 11 February 2020 (2020-02-11),<br>pp. 253-255 | 1-9 |
| PX | WANG, Manli et al. "Remdesivir and Chloroquine Effectively Inhibit the Recently Emerged Novel Coronavirus (2019-nCoV) in Vitro,"<br>*Cell Research,* Vol. 30, 04 February 2020 (2020-02-04),<br>pp. 269-271 | 1-9 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **22 October 2020** | **06 November 2020** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/093989**

**C. DOCUMENTS CONSIDERED TO BE RELEVANT**

| Category* | Citation of document, with indication, where appropriate, of the relevant passages | Relevant to claim No. |
|---|---|---|
| PX | TOURET, Franck et al. "Of Chloroquine and COVID-19," *Antiviral Research,* Vol. 177, 05 March 2020 (2020-03-05), pp. 1 and 2 | 1-9 |
| PX | LIU, Jia et al. "Hydroxychloroquine, a Less Toxic Derivative of Chloroquine, is Effective in Inhibiting SARS-CoV-2 Infection in Vitro," *Cell Discovery,* Vol. 16, No. 6, 18 March 2020 (2020-03-18), pp. 1-4 | 1-9 |
| PX | COLSON, Philippe et al. "Chloroquine and Hydroxychloroquine as Available Weapons to Fight COVID-19" *International Journal of Antimicrobial Agents,* Vol. 55, No. 4, 04 March 2020 (2020-03-04), pp. 1-3 | 1-9 |
| X | CN 103027915 A (INSTITUTE OF BASIC MEDICAL SCIENCES, CHINESE ACADEMY OF MEDICAL SCIENCES et al.) 10 April 2013 (2013-04-10) description, paragraphs [0011]-[0020] | 6-9 |
| X | CN 1612735 A (Charous Lauren) 04 May 2005 (2005-05-04) claims 1-56 | 6-9 |
| A | BIOT, Christophe et al. "Design and Synthesis of Hydroxyferroquine Derivatives with Antimalarial and Antiviral Activities," *Journal of Medicinal Chemistry,* Vol. 49, No. 9, 08 April 2006 (2006-04-08), pp. 2845-2849 | 1-9 |
| A | WO 2015157223 A1 (UNIVERSITY OF MARYLAND, BALTIMORE) 15 October 2015 (2015-10-15) claims 1-24 | 1-9 |
| A | HAN, Dong P. et al. "Development of a Safe Neutralization Assay for SARS-CoV and Characterization of S-glycoprotein," *Virology,* Vol. 326, 31 December 2004 (2004-12-31), pp. 140-149 | 1-9 |
| A | BARNARD, Dale, L. et al. "Evaluation of Immunomodulators, Interferons and Known in Vitro SARS-CoV Inhibitors for Inhibition of SARS-CoV Replication in BALB/c Mice," *Antiviral Chemistry & Chemotherapy,* Vol. 17, 31 December 2006 (2006-12-31), pp. 275-284 | 1-9 |

Form PCT/ISA/210 (second sheet) (January 2015)

**INTERNATIONAL SEARCH REPORT**

International application No.
**PCT/CN2020/093989**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed:
     - ☑ in the form of an Annex C/ST.25 text file.
     - ☐ on paper or in the form of an image file.
   - b. ☐ furnished together with the international application under PCT Rule 13*ter*.1(a) for the purposes of international search only in the form of an Annex C/ST.25 text file.
   - c. ☐ furnished subsequent to the international filing date for the purposes of international search only:
     - ☐ in the form of an Annex C/ST.25 text file (Rule 13*ter*.1(a)).
     - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.
**PCT/CN2020/093989**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **5, 8-9**
because they relate to subject matter not required to be searched by this Authority, namely:

[1] Claims 5, 8 and 9 set forth a method of treating and/or preventing a disease or viral infection in a mammal in need or a method of inhibiting the replication or propagation of SARS-CoV-2 in a mammal in need, comprising a method for treatment of a human or animal body, which does not comply with PCT Implementing Rule 39.1(iv). A search for claims 5, 8 and 9 is made on the basis of the following modifications: the use of hydroxychloroquine shown in formula I or chloroquine shown in formula II, or a geometric isomer thereof or a pharmaceutically acceptable salt thereof and/or a solvent compound thereof and/or a hydrate thereof or a therapeutically and/or prophylactically effective amount of a pharmaceutical composition comprising the above compounds in the preparation of a drug for the treatment and/or prophylaxis of a disease or viral infection or the inhibition of SARS-CoV-2 replication or propagation.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2020/093989**

| Patent document cited in search report | Publication date (day/month/year) | Patent family member(s) | Publication date (day/month/year) |
|---|---|---|---|
| CN 111658648 A | 15 September 2020 | None | |
| CN 103027915 A | 10 April 2013 | WO 2013044871 A1 | 04 April 2013 |
| | | CN 103687599 A | 26 March 2014 |
| CN 1612735 A | 04 May 2005 | MX PA04004393 A | 12 September 2005 |
| | | EP 1450803 A4 | 03 September 2008 |
| | | IL 161821 D0 | 20 November 2005 |
| | | CA 2466338 A1 | 15 May 2003 |
| | | AU 2002363443 B2 | 16 February 2006 |
| | | HK 1075619 A1 | 20 July 2007 |
| | | US 2004167162 A1 | 26 August 2004 |
| | | WO 03039546 A1 | 15 May 2003 |
| | | CA 2466338 C | 12 January 2010 |
| | | EP 1450803 A1 | 01 September 2004 |
| | | JP 2005512998 A | 12 May 2005 |
| | | CN 1289086 C | 13 December 2006 |
| WO 2015157223 A1 | 15 October 2015 | US 2017027975 A1 | 02 February 2017 |
| | | US 10434116 B2 | 08 October 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010078808